# EUROPEAN PATENT APPLICATION

(11) **EP 4 421 066 A1**
(43) Date of publication of application: **28.08.2024**
(21) Application number: 22909712.6
(22) Date of filing: 02.12.2022
(51) Int. Cl.: C07D 219/12, A61K 31/473, A61P 31/12, A61P 35/00

(54) **INNATE IMMUNE-ACTIVATING DRUG AND USE THEREOF**

(30) Priority: 25.12.2021 CN 202111605896
(71) Applicant: Luoda Biopharma (Suzhou) Co., Ltd., Suyhou, Jiangsu 215125 (CN)
(72) Inventor: HOU, Fajian, Suzhou, Jiangsu 215125 (CN)
(74) Representative: Sun, Yiming
(86) International application number: PCT/CN2022/136399
(87) International publication number: WO 2023/116397

(57) **Abstract**

Disclosed is an innate immune-activating drug, which has broad-spectrum antiviral activity, immune adjuvant activity, promotive immunotherapy and anti-tumor activity. Specifically, disclosed are use of a compound of formula I or a tautomer, a mesomer, a racemate, an enantiomer, a diastereoisomer or a mixture thereof, or a pharmaceutically acceptable salt thereof, or a prodrug thereof in the preparation of an innate immune-activating drug. The compound can activate innate immunity, induce generation of type I interferon, significantly inhibit virus infection, and promote immune function and anti-tumor.

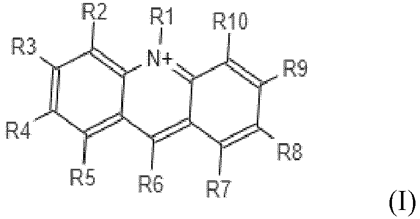

## Description

### Technical Field

The present invention belongs to the field of medicine, and particularly relates to an innate immune-activating drug having broad-spectrum antiviral activity and used for immunotherapy, anti-tumor and immune adjuvants and use thereof.

### Background Art

Viral infections can cause a wide range of diseases, and innate immune is the first natural line of defense of a host's immune system against pathogenic microorganisms. An antiviral innate immune response induces host cells to generate interferon to inhibit reproduction and amplification of viruses which can escape the innate immune response during evolution. Thus, the generation of interferon by inducing the innate immune response provides a new idea for antiviral therapy.

Currently, recombinantly expressed interferon is widely applied to the treatment of viral infections, inflammation, immune dysfunction and the like, but it is a protein peptide that lacks posttranslational modification, is structurally unstable, cannot be taken orally and has high requirements for storage conditions. It can only be administered by injection with a single mode of administration. In addition, its short half-life period and high concentration will cause serious toxic and side effects. The prominent drawbacks of interferons recombinantly expressed in E. coli are lack of glycosylation modification and restricted viability; however, glycosylation-containing interferons purified by eukaryotic system expression are high in cost and drug-forming price.

In summary, there is an urgent need in the art to develop a new drug having an ability to induce interferon generation.

In addition, the immune system of an organism can perform the function of immune surveillance against tumors; on one hand, the immune system can recognize and specifically remove malignant cells through an immune mechanism to resist the generation and development of tumor; on the other hand, malignant cells may escape immune surveillance of the organism through various mechanisms and rapidly proliferate in the organism, so that tumors are formed. The development and regression of tumors depend on the action results of these two aspects. Antibodies as therapeutic drugs for tumors have excellent therapeutic effects due to their strong specificity. In addition, the quantity of antibodies generated in animals is low during generation of antibodies due to factors such as slow and poor immune response of the organism to antigens, thus limiting their use and requiring the action of immune adjuvants. The immune adjuvant, abbreviated as adjuvant, refers to an auxiliary substance injected into a body before or at the same time as an antigen, which can enhance an immune response of an organism to the antigen or change the type of an immune response. Immunobiological effects of adjuvants include prolongation of retention time of an antigen in an organism, enhancement of antigen processing and presentation, and enhancement and amplification of an immune response. Immune adjuvants are widely applied because they have an effect of enhancing the immune response. The immune adjuvants should have the following characteristics: the adjuvants should have small side effects on specific animals; the effect of the adjuvants should be long-lasting and stable; the generation cost should be as low as possible; the immune response generated should be appropriate, and it is preferable to induce an organism to produce cellular immune response or humoral immune response which should meet protection requirements. Therefore, development of a new immune adjuvant having high efficiency and low toxicity and capable of enhancing an immune level, cytokine secretion and immunological memory of an organism is one of hot spots in current research.

Therefore, drugs for promoting tumor immunotherapy and serving as immune adjuvants are to be developed in the art.

### DISCLOSURE

The present invention is to solve the current technical problem that there is no new drug that can effectively enhance the innate immune of a human body, and to provide an innate immune-activating drug that can not only induce interferon generation against viral infections, but also promote tumor immunotherapy while serving as an immune adjuvant.

In one aspect of the present invention, provided is use of a compound of formula I or a tautomer, a mesomer, a racemate, an enantiomer, a diastereoisomer or a mixture thereof, or a pharmaceutically acceptable salt thereof, or a prodrug thereof for preparing an innate immune-activating drug, 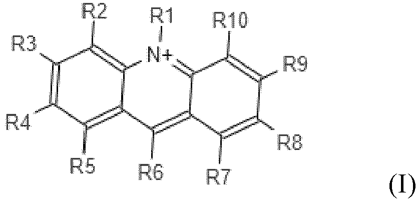 wherein R1, R2, R3, R4, R5, R6, R7, R8, R9 and R10 are each independently selected from hydrogen, halogen, alkyl, cycloalkyl, aryl, heteroaryl, heterocyclyl, alkenyl, alkynyl, amino, hydroxy, hydrosulfuryl, carboxy, alkoxy, cycloalkoxy, haloalkyl, cyano, thioalkyl, sulpho, sulfuryl, sulfoxide group and phosphate group, and the alkyl, cycloalkyl, alkoxy, cycloalkoxy, amino, heterocyclyl, aryl or heteroaryl may be substituted with one or more substituents selected from one or more of hydroxyl, halogen, alkyl, alkoxy, cycloalkyl, cycloalkoxy, heterocyclyl, aryl, haloaryl, haloalkyl, heteroaryl, alkenyl, alkynyl, amino, sulfydryl, carboxy, ester group, alkoxycarbonyl, acyloxy, acylamino, carbamido, alkylsulfonyl, aryl sulfonyl, cyano, nitro, nitroso, thiocyanato, isothiocyanato, thioalkyl, sulfonic group, phosphoryl group, phosphonic acid group, alkyl phosphate group, alkyl phosphonic acid group, aryl phosphate group and aryl phosphonic acid group, or null; the heterocyclyl includes at least one N atom, or includes 1 or 2 or 3 heteroatoms selected from N, S and O.

Wherein the term "substituted" means that one or more (preferably 1, 2, 3, 4, 5, 6, 7 or 8) hydrogen atoms on a ring or group are substituted with a substituent selected from the group consisting of hydroxyl, halogen, alkyl, alkoxy, cycloalkyl, cycloalkoxy, heterocyclyl, aryl, haloaryl, haloalkyl, heteroaryl, alkenyl, alkynyl, amino, sulfydryl, carboxy, ester group, alkoxycarbonyl, acyloxy, acylamino, carbamido, alkylsulfonyl, aryl sulfonyl, cyano, nitro, nitroso, thiocyanato, isothiocyanato, thioalkyl, sulfonic group, phosphoryl group, phosphonic acid group, alkyl phosphate group, alkyl phosphonic acid group, aryl phosphate group and aryl phosphonic acid group.

The heteroaryl has 1-4 (preferably 1, 2, 3 or 4) heteroatoms selected from N, O and S on the heterocycle.

In another preferred embodiment, R1, R2, R5, R7 and R10 are each independently hydrogen, substituted or unsubstituted C1-C10 alkyl, and substituted or unsubstituted C3-C12 cycloalkyl.

In another preferred embodiment, R1, R2, R5, R7 and R10 are each independently hydrogen, substituted or unsubstituted C1-C8 alkyl, and substituted or unsubstituted C3-C8 cycloalkyl.

In another preferred embodiment, R1, R2, R5, R7 and R10 are each independently hydrogen, substituted or unsubstituted C1-C6 alkyl, and substituted or unsubstituted C3-C8 cycloalkyl.

In another preferred embodiment, R1, R2, R5, R7 and R10 are each independently hydrogen, substituted or unsubstituted C1-C4 alkyl, and substituted or unsubstituted C3-C8 cycloalkyl.

In another preferred embodiment, R1, R2, R5, R7 and R10 are each independently hydrogen, methyl, ethyl, propyl or butyl.

In another preferred embodiment, R3 and R4 are each independently hydrogen, substituted or unsubstituted C1-C8 alkoxy, and substituted or unsubstituted C1-C8 alkylthio.

In another preferred embodiment, R3 and R4 are each independently hydrogen, substituted or unsubstituted C1-C6 alkoxy, and substituted or unsubstituted C1-C6 alkylthio.

In another preferred embodiment, R3 and R4 are each independently hydrogen, substituted or unsubstituted C1-C4 alkoxy, and substituted or unsubstituted C1-C4 alkylthio.

In another preferred embodiment, R3 and R4 are each independently hydrogen, substituted or unsubstituted C1-C2 alkoxy, and substituted or unsubstituted C1-C2 alkylthio.

In another preferred embodiment, R6 is substituted or unsubstituted amino, substituted or unsubstituted heterocyclyl, heterocyclic alkyl or an alkyl heterocyclyl.

In another preferred embodiment, R8 is halogenated alkyl.

In another preferred embodiment, R9 is hydrogen, halogen or amino.

In another preferred embodiment, the compound of formula I includes:

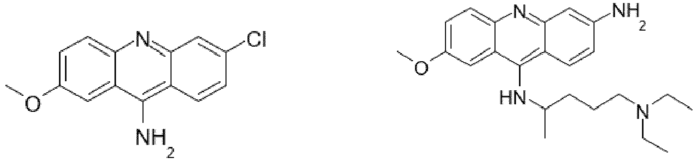

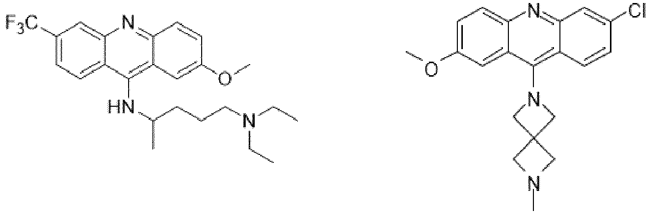

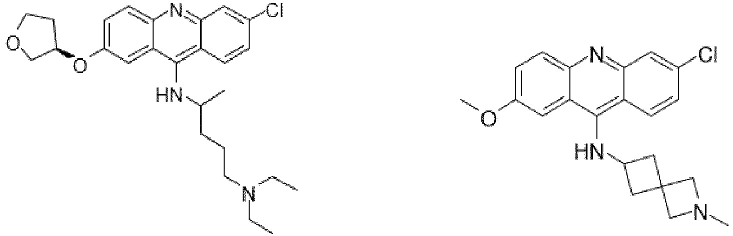

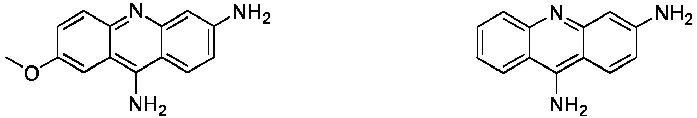

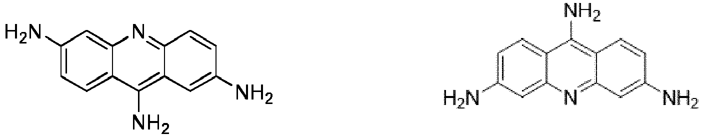

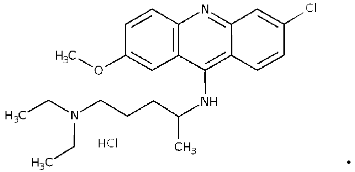

Preferably, the pharmaceutically acceptable salt of the compound of formula I includes:

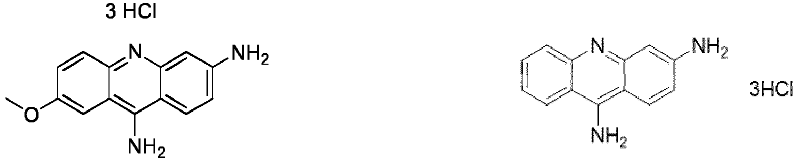

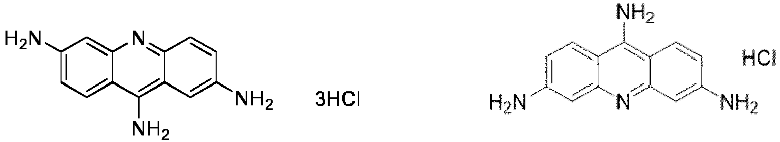

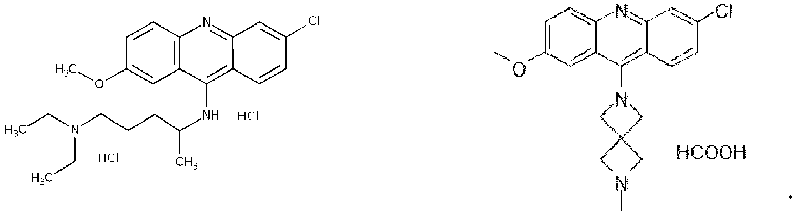

Preferably, the innate immune-activating drug includes:
(a) a broad-spectrum antiviral drug;
(b) an anti-tumor drug;
(c) a drug for preventing and/or treating cancer;
(d) a drug for treating or preventing type I interferon-related diseases;
(e) an immune adjuvant; and/or
(f) a cytokine inducer.

In another preferred embodiment, the cytokine is selected from the group consisting of interferon, tumor necrosis factor and interleukin, or a combination thereof.

In another preferred embodiment, the interferon is selected from the group consisting of type I interferon, type II interferon and type III interferon, or a combination thereof.

In another preferred embodiment, the interferon is selected from the group consisting of IFNβ (interferon-β), IFNA (interferon-λ) and TNFα (tumor necrosis factor-α), or a combination thereof.

In another preferred embodiment, the interleukin is selected from the group consisting of IL6 (interleukin-6) and IL1α (interleukin-1α), or a combination thereof.

In another preferred embodiment, the cytokines include cytokines in blood, serum or plasma.

In another preferred embodiment, the cytokine inducer increases the level or content of cytokines.

In another preferred embodiment, the blood includes peripheral blood.

In another preferred embodiment, the inducer includes:
(i) inducing expression of cytokines; and/or
(ii) inducing an elevated content of cytokines.

In another preferred embodiment, the inducer includes inducing elevation of cytokines in peripheral blood.

In another preferred embodiment, the adjuvant includes an antibody adjuvant.

In another preferred embodiment, the immune adjuvant is used to enhance the immune titer of an antibody.

In another preferred embodiment, the immune adjuvant is used to promote the generation of antibodies or used as an antibody-producing promoter.

In another preferred embodiment, the antibodies include a serum antibody.

In another preferred embodiment, the antigen against which the antibody is directed includes a protein antigen.

In another preferred embodiment, the protein antigen includes an albumin antigen.

In another preferred embodiment, the albumin antigen includes an ovalbumin antigen.

In another preferred embodiment, the immune adjuvant is used to prepare antibodies, vaccines, immunotherapeutic drugs and/or immune activators.

In another preferred embodiment, the antibody is selected from the group consisting of IgG, IgM, or a combination thereof.

In another preferred embodiment, the IgG includes IgG1.

In another preferred embodiment, the vaccine includes a tumor vaccine.

In another preferred embodiment, the type I interferon-related diseases refer to diseases ameliorated, treated or prevented by increasing the level of type I interferon in human bodies.

In another preferred embodiment, the type I interferon (IFN)-related diseases include: viral infections, and multiple sclerosis and/or chronic granulocytic leukemia.

In another preferred embodiment, the viruses include DNA viruses and RNA viruses.

In another preferred embodiment, the DNA viruses include: HSV (Herpes Simplex Virus), HBV (Hepatitis B Virus), HAV (Hepatitis A Virus), HPV (Human Papilloma Virus) and/or EBV (Epstein-Barr Virus).

In another preferred embodiment, the RNA viruses include: VSV (Vesicular Stomatitis Virus), HCV (Hepatitis C Virus), EMCV (Encephalomyocarditis Virus), EBOV (Ebola Virus), HIV (Human Immunodeficiency Virus), and/or ZIKV (Zika Virus).

In another aspect of the present invention, further provided is a method for inducing generation of type I interferon in a subject, wherein the method includes a step of: contacting a subject with a compound of formula I to induce generation of type I interferon in the subject.

In another preferred embodiment, the subject is a cell.

In another preferred embodiment, the cell includes: Vero cells, THP-1 cells, HEK293 cells, HEK293T cells, and/or A549 cells.

In another preferred embodiment, the method is an in vitro non-therapeutic method.

In another preferred embodiment, the method includes a step of: cultivating the subject in a system containing a compound of formula I to induce generation of interferon in the subject.

In another preferred embodiment, the concentration of the compound of formula I in the system containing the compound of formula I is ≥1 µM; preferably, ≥3 µM.

In another preferred embodiment, the incubation time is 10 min to 48 h.

In another preferred embodiment, the type I interferon includes IFNβ.

In another aspect of the present invention, further provided is a method for inhibiting viral infections, wherein the method includes a step of:
contacting a subject with a compound of formula I to inhibit infection of the subject by a virus.

In another preferred embodiment, the subject is a cell.

In another preferred embodiment, the cell includes: Vero cells, THP-1 cells, HEK293 cells, HEK293T cells, and/or A549 cells.

In another preferred embodiment, the viruses include DNA viruses and RNA viruses.

In another preferred embodiment, the DNA viruses include: HSV (Herpes Simplex Virus), HBV (Hepatitis B Virus), HAV (Hepatitis A Virus), HPV (Human Papilloma Virus) and/or EBV (Epstein-Barr Virus) and the like.

In another preferred embodiment, the RNA viruses include: VSV (Vesicular Stomatitis Virus), HCV (Hepatitis C Virus), EMCV (Encephalomyocarditis Virus), EBOV (Ebola Virus), HIV (Human Immunodeficiency Virus), and/or Zika virus (ZIKV) and the like.

In another preferred embodiment, the method is an in vitro non-therapeutic method.

In another preferred embodiment, the method includes a step of: cultivating the subject in a system containing a compound of formula I to induce generation of interferon in the subject.

In another aspect of the present invention, further provided is a method for increasing the level of type I interferon in a human body, wherein the method includes a step of:
administering to a subject in need thereof a compound of formula I, or a pharmaceutically acceptable salt thereof.

In another preferred embodiment, the subject is a mammal; preferably, the subject is selected from the group consisting of humans, mice and rats.

In another aspect of the present invention, further provided is broad-spectrum antiviral pharmaceutical composition, wherein the pharmaceutical composition contains: a compound of formula I or a tautomer, a mesomer, a racemate, an enantiomer, a diastereoisomer or a mixture thereof, or a pharmaceutically acceptable salt thereof, or a prodrug thereof, and one or more pharmaceutically acceptable carriers, diluents or excipients.

In another aspect of the present invention, further provided is an immune composition, wherein the immune composition contains: a compound of formula I or a tautomer, a mesomer, a racemate, an enantiomer, a diastereoisomer or a mixture thereof, or a pharmaceutically acceptable salt thereof, or a prodrug thereof; and an antigen.

In another preferred embodiment, the antigen includes a protein antigen.

In another preferred embodiment, the protein antigen includes an albumin antigen.

In another preferred embodiment, the albumin antigen includes an ovalbumin antigen.

In another aspect of the present invention, further provided is an anti-tumor pharmaceutical composition, wherein the pharmaceutical composition contains: a compound of formula I or a tautomer, a mesomer, a racemate, an enantiomer, a diastereoisomer or a mixture thereof, or a pharmaceutically acceptable salt thereof, or a prodrug thereof, and one or more pharmaceutically acceptable carriers, diluents or excipients.

In another aspect of the present invention, further provided is a composition, wherein the composition comprises:
(a) a first active ingredient, which is a compound of formula I or a tautomer, a mesomer, a racemate, an enantiomer, a diastereoisomer or a mixture thereof, or a pharmaceutically acceptable salt thereof, or a prodrug thereof; and
(b) a therapeutically effective amount of second active ingredient, which includes: an immunotherapeutic activator, a type I interferon modulator, or an antiviral agent.

In another aspect of the present invention, further provided is a kit, wherein the kit comprises:
(A) a first formulation containing a first active ingredient which is a compound of formula I or a tautomer, a mesomer, a racemate, an enantiomer, a diastereoisomer or a mixture thereof, or a pharmaceutically acceptable salt thereof, or a prodrug thereof; and
(B) a second formulation containing a second active ingredient which includes: an immunotherapeutic activator, a type I interferon modulator, or an antiviral agent.

In another preferred embodiment, the immunotherapeutic activator includes a PD-1 or PD-L1 antibody, and the compound of formula I of the present invention and the PD-1 or PD-L1 antibody can used together to exert a synergistic effect, increase an inhibitory effect on tumour growth significantly, and lenghthen a survival period obviously.

In another aspect of the present invention, further provided is use of the above-mentioned composition or kit for preparing innate immune-activating drugs, wherein the drugs include:
(a) a broad-spectrum antiviral drug;
(b) an anti-tumor drug;
(c) a drug for preventing and/or treating cancer;
(d) a drug for treating or preventing type I interferon-related diseases;
(e) an immune adjuvant; and/or
(f) a cytokine inducer.

The innate immune-activating drug of the present invention includes the main advantages that:
(a) The present invention discovers that the compound of formula I exhibits a high ability to induce the expression of interferon (such as IFNβ).
(b) The present invention discovers that the compound of formula I has an excellent ability to inhibit viral infection and antiviral activity.
(c) The present invention discovers that the compound of formula I can induce generation of cytokines efficiently and safely so as to be used to treat cytokine-related diseases such as tumors.
(d) The present invention discovers that the compound of formula I can be used as an immune adjuvant to increase the immune titer of an antibody significantly and promote the generation of the antibody, so as to be used to prepare antibodies, vaccines, immunotherapeutic drugs and immune activators and improve the application value of the antibody.
(e) The compound described herein has high efficacy, good druggability and low side effects.

It should be understood that each of the above-mentioned technical characteristics of the present invention and each of the technical characteristics specifically described below (for example, in the embodiments) may be combined with each other within the scope of the present invention to form new or preferred technical solutions. The details will not be repeated here because of space limitation.

### DESCRIPTION OF DRAWINGS

The present invention is described below in detail in conjunction with the accompanying drawings and specific embodiments.
FIG. 1 is a graph showing transcriptional activation results of type I interferon and interleukin-6 in HEK293T cells of Example 1 of the present invention after 12 hours of treatment with compounds LD001-LD004 at a final concentration of 10 µM, respectively;
FIG. 2 is a graph showing transcriptional activation results of type I interferon and interleukin-6 in HeLa cells of Example 1 of the present invention after 12 hours of treatment with compounds LD001-LD004 at a final concentration of 10 µM, respectively;
FIG. 3 is a graph showing transcriptional activation results of type I interferon and interleukin-6 in A549 cells of Example 1 of the present invention after 12 hours of treatment with compounds LD001-LD004 at a final concentration of 10 µM, respectively;
FIG. 4 is a graph showing transcriptional activation results of type I interferon and interleukin-6 in HEK293T cells of Example 1 of the present invention after 12 hours of treatment with compounds LD005-LD011 at a final concentration of 10 µM, respectively;
FIG. 5 is a graph showing transcriptional activation results of type I interferon and interleukin-6 in PEM cells of Example 1 of the present invention after 12 hours of treatment with compounds LD005-LD011 at a final concentration of 10 µM, respectively;
FIG. 6 is an inhibition curve of LD002 of Example 2 of the present invention for VSV-GFP;
FIG. 7 is an inhibition curve of LD002 of Example 2 of the present invention for H1N1-IAV;
FIG. 8 is an inhibition curve of LD002 of Example 3 of the present invention for HSV;
FIG. 9 is a graph showing the results of ELISA assay for inducing interferon generation in mice in vivo with the compound LD002 of Example 4 of the present invention;
FIG. 10 is a graph showing the experimental results of melanoma growth inhibition with the compound LD002 of Example 5 of the present invention administered alone;
FIG. 11 is a graph showing the experimental results of melanoma growth inhibition with the compound LD002 of Example 5 of the present invention administered in combination with another immunotherapeutic activator, anti-PD-L1;
FIG. 12 is a graph showing the results of the ELISA method of Example 6 of the present invention for testing the compounds of the present invention for immune adjuvant activity.

### MODE FOR DISCLOSURE

After extensive and in-depth research, the inventor has discovered that a class of ternary heterocyclic compounds are capable of activating a host's innate immune and inducing different cells to express or generate cytokines, such as type I interferon IFNβ. Furthermore, such compounds have a broad-spectrum inhibitory effect (dose-related) on RNA virus (such as VSV) and DNA virus (such as HSV) infections. Thus, provided is a new drug with the ability to induce cells to generate IFNβ and a potential broad-spectrum antiviral effect in clinical therapy. It is unexpectedly discovered that the compound of formula I described herein has an excellent induction effect on cytokines and is used to treat diseases such as tumors. Also, the compound of formula I described herein can also be used as an immune adjuvant to increase the immune titer of an antibody significantly and promote the generation of the antibody, so as to be used to prepare antibodies, vaccines, immunotherapeutic drugs and immune activators and to improve the application value of the antibody.

### Innate immune

A host's innate immune is the first line of defense of the immune system against pathogenic microorganisms. A virus in nature is a pathogen capable of causing various diseases, and the antiviral innate immune response of host cells can induce interferon generation to inhibit reproduction and amplification of the virus. The antiviral innate immune response is mediated by a signal transduction pathway, and previous research reveals how the innate immune system recognizes virus invasion and rapidly triggers an immune defensive function to achieve virus clearance effectively. This is important research in the field of life sciences and medicine, which provides a theoretical basis for the design of relevant drugs and brings new hope for prevention and treatment of viral infections. After detecting pathogenic microorganisms, host cells induce generation of interferons, proinflammatory factors and chemokines through an innate immune signalling pathway, and on one hand, these effectors can inhibit pathogen replication to play a role in controlling pathogen proliferation rapidly; on the other hand, these cytokines can mobilize other immune cells of the host, including antigen-presenting cells in the adaptive immune system as well as T and B lymphocytes, to activate adaptive immunity. Besides, the induced activation of the innate immune response has an anti-tumor effect and enhances the effects of radiotherapy and chemotherapy.

The innate immune signalling pathways begin with recognition of relevant molecular patterns of pathogenic microorganisms by pattern recognition receptors in host cells. The innate immune signalling pathways having been identified in relation to viral infection mainly include a RLR-MAVS signalling pathway for recognizing RNA viruses and a cGAS-STING signalling pathway for recognizing DNA viruses in cytoplasms, as well as a TLRs signalling pathway. After activation, both signalling pathways, RLRs and cGAS, recruit and activate TBK1 through downstream transduction proteins, TBK1 phosphorylates a transcription factor IRF3, which leads to IRF3 translocation into the nucleus, to induce generation of type I interferon, and an effect of virus suppression is achieved ultimately. Some viruses have also acquired some mechanisms for escaping the innate immune response over a long period of evolution.

Innate immune is of important instructive significance for both research of vaccines and development of drugs since innate immune plays an important role in every link of life activities of an organism.

### Terminology

### Active ingredient

The term "compound of the present invention" as used herein refers to a compound of formula I. The term also includes various crystal forms, pharmaceutically acceptable salts, isomers, hydrates, solvates or prodrugs of the compound of formula I.

Wherein the term "pharmaceutically usable salt" or "pharmaceutically acceptable salt" refers to a salt suitable for use as a drug formed from the compound of the present invention and an acid or base. Pharmaceutically acceptable salts include inorganic and organic salts. A preferred class of salts are those formed from the compound of the present invention and acids. Acids suitable for formation of salts include, but are not limited to: inorganic acids, such as hydrochloric, hydrobromic, hydrofluoric, sulfuric, nitric and phosphoric acids; organic acids, such as formic, acetic, trifluoroacetic, propionic, oxalic, malonic, succinic, fumaric, maleic, lactic, malic, tartaric, citric, picric, benzoic, methanesulphonic, ethanesulfonic, p-toluenesulphonic, benzenesulphonic, naphthalene sulphonic acids; and amino acids, such as proline, phenylalanine, aspartic acid and glutamic acid. Another preferred class of salts are those formed from the compound of the present invention and bases, for example, alkali metal salts (e.g., sodium or potassium salts), alkaline-earth metal salts (e.g., magnesium or calcium salts), ammonium salts (e.g., lower alkanolammonium salts and other pharmaceutically acceptable amine salts), such as methylamine salts, ethylamine salts, propylamine salts, dimethylamine salts, trimethylamine salts, diethylamine salts, triethylamine salts, tertiary butylamine salts, ethylenediamine salts, hydroxyethylamine salts, dihydroxyethylamine salts, trihydroxyethylamine salts, as well as amine salts formed from morpholine, piperazine and lysine, respectively.

The compound of formula I described herein may be converted into its pharmaceutically acceptable salts by conventional methods. For example, a solution of a corresponding acid can be added to a solution of the above-mentioned compound, and a corresponding salt of the compound described herein is obtained by removing the solvent after the salt is completely formed.

The term "prodrug" as used herein includes a prodrug, which may be biologically active or inactive itself, which, when administered by an appropriate method, is metabolised or undergoes a chemical reaction, and is converted in a human body to a class of compound of formula I, or to a salt or solution consisting of one compound of formula I. The predrugs include (but are not limited to) the forms of carboxylic acid esters, carbonate esters, phosphate esters, nitrate esters, sulphate esters, sulfone esters, sulfoxide esters, amino compounds, carbamates, azoic compounds, phosphamides, glucosides, ethers, acetals and the like of the compound.

### First active ingredient

The first active ingredient of the present invention is the compound of formula I, or a pharmaceutically acceptable salt thereof, or an optical isomer thereof, or a racemate thereof, or a solvate thereof, or a prodrug thereof.

As used herein, the "compound of the present invention", "compound of formula I of the present invention" and "compound of formula I" are interchangeable, which refers to a compound of formula I, or a tautomer, a mesomer, a racemate, an enantiomer, a diastereoisomer or a mixture thereof, or a pharmaceutically acceptable salt thereof, or a prodrug thereof. It should be understood that the term also includes mixture of the above-mentioned components.

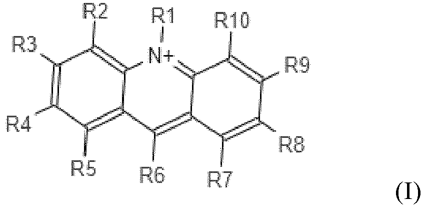

In formula I, R1, R2, R3, R4, R5, R6, R7, R8, R9 and R10 are each independently selected from hydrogen, halogen, alkyl, cycloalkyl, aryl, heteroaryl, heterocyclyl, alkenyl, alkynyl, amino, hydroxy, hydrosulfuryl, carboxy, alkoxy, cycloalkoxy, haloalkyl, cyano, thioalkyl, sulpho, sulfuryl, sulfoxide group and phosphate group, and the alkyl, cycloalkyl, alkoxy, cycloalkoxy, amino, heterocyclyl, aryl or heteroaryl may be substituted with one or more substituents selected from one or more of hydroxyl, halogen, alkyl, alkoxy, cycloalkyl, cycloalkoxy, heterocyclyl, aryl, haloaryl, haloalkyl, heteroaryl, alkenyl, alkynyl, amino, sulfydryl, carboxy, ester group, alkoxycarbonyl, acyloxy, acylamino, carbamido, alkylsulfonyl, aryl sulfonyl, cyano, nitro, nitroso, thiocyanato, isothiocyanato, thioalkyl, sulfonic group, phosphoryl group, phosphonic acid group, alkyl phosphate group, alkyl phosphonic acid group, aryl phosphate group and aryl phosphonic acid group, or null; the heterocyclyl includes at least one N atom, or includes 1 or 2 or 3 heteroatoms selected from N, S and O.

Preferred compounds of the present invention include:

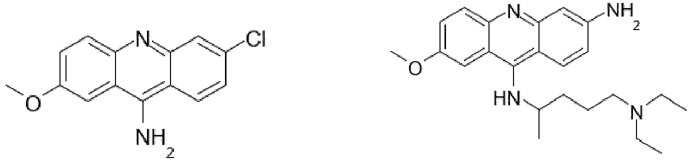

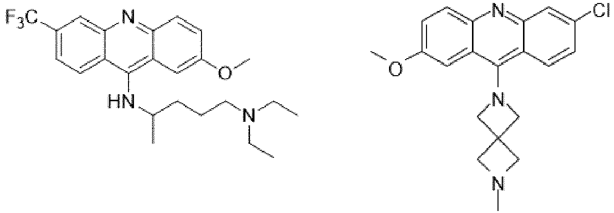

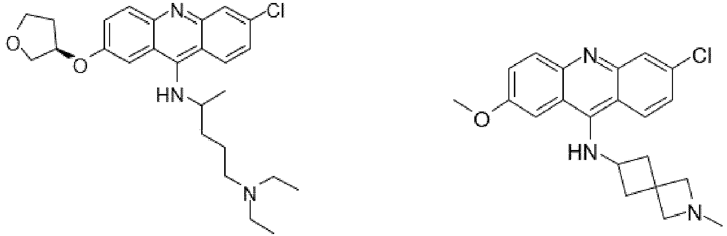

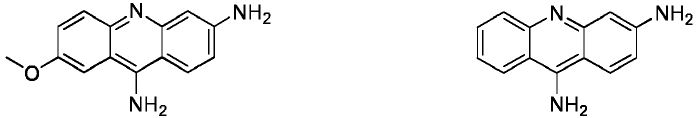

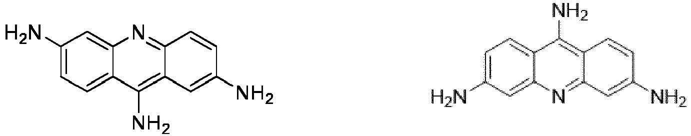

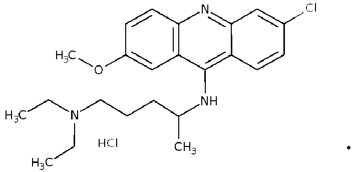

Pharmaceutical salts of the preferred compounds of the present invention include:

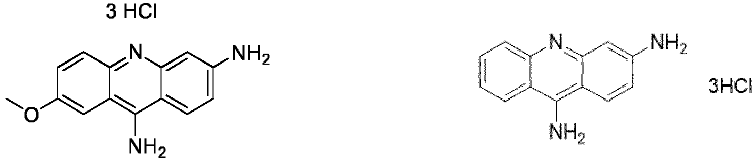

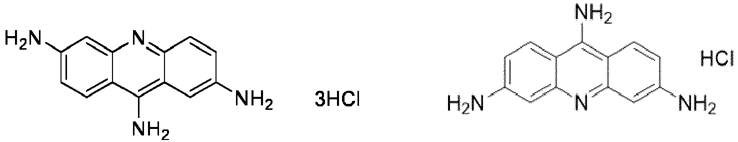

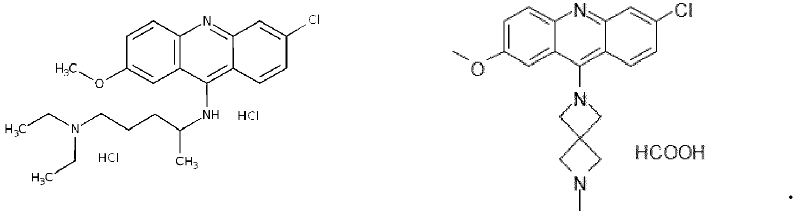

### Second active ingredient

The second active ingredient of the present invention is an antiviral, anti-tumor, immunomodulatory and immune-adjuvant drug, which can be used in combination with the PD-1 or PD-L1 antibody for immunotherapy.

The term "PD-L1" as used herein refers to programmed cell death-ligand 1, which is an important immunosuppressive molecule, and PD-L1 will be expressed on the surface of tumors and binds to PD-1 on the surface of T-cells to inhibit T-cell proliferation and cytokine secretion, regulate lymphocyte activation negatively and participate in immune escape of tumors.

### Pharmaceutical compositions and methods for administration

As the compound of the present invention has an excellent ability to induce type I interferon, the compound of the present invention and various crystal forms thereof, pharmaceutically acceptable inorganic or organic salts, hydrates or solvates, as well as pharmaceutical compositions containing the compound of the present invention as the main active ingredient, can be used to treat, prevent and alleviate type I interferon-related diseases (particularly those that can be ameliorated, treated or prevented by regulating the levels of interferon in vivo, such as increasing the levels of interferon). According to the prior art, the compound of the present invention can be used to treat the following diseases: virus infection, multiple sclerosis, systemic lupus erythematosus and chronic granulocytic leukemia and the like.

As used herein, the terms "induce generation of interferons", "induce generation of type I interferon", "induce generation of type I interferon" or "inducing generation of interferons" are interchangeable and are intended to include: improving the expression, transcription or protein content level of type I interferon or a protein, cytokine and the like associated with type I interferon.

The pharmaceutical compositions of the present invention include a safe and effective amount of a compound of the present invention or a pharmacologically acceptable salt thereof and a pharmacologically acceptable excipient or carrier. The term "safe and effective amount" refers to an amount of the compound that is sufficient for significant improvement of the condition without causing serious side effects. Typically, the pharmaceutical compositions contain 1 -2000 mg of the compound/agent of the present invention, and preferably 10-500 mg of the compound/agent of the present invention. Preferably, "a dose" is one capsule or tablet.

The term "pharmaceutically acceptable carrier" refers to one or more compatible solid or liquid fillers or gel substances which are suitable for use in human and must have sufficient purity and sufficiently low toxicity. The term "compatibility" herein refers to the ability of the components in a composition to be admixed with the compound of the present invention and with each other without significantly reducing the efficacy of the compound. Some examples of pharmaceutically acceptable carriers are cellulose and derivatives thereof (such as sodium carboxymethylcellulose, sodium ethylcellulose, cellulose acetate and the like), gelatin, talc, solid lubricants (such as stearic acid, magnesium stearate), calcium sulphate, vegetable oils (such as soya bean oil, sesame oil, peanut oil, olive oil and the like), polyols (such as propylene glycol, glycerol, mannitol, sorbitol and the like), emulsifiers (such as Tween^{®}), wetting agents (such as sodium dodecyl sulphate), coloring agents, flavoring agents, stabilizers, antioxidants, preservatives, pyrogen-free water and the like.

There is no special limitation to the modes of administration of the compound or pharmaceutical composition of the present invention, and representative modes of administration include (but are not limited to): oral, intratumoral, rectal, parenteral (intravenous, intramuscular or subcutaneous) and topical administration.

Solid dosage forms for oral administration include capsules, tablets, pills, powders and granules. Among these solid dosage forms, the active compound is mixed with at least one conventional inert excipient (or carrier), such as sodium citrate or dicalcium phosphate, or mixed with the following ingredients: (a) fillers or bulking agents, e.g., starch, lactose, sucrose, glucose, mannitol, and silicic acid; (b) binders, e.g., hydroxymethyl cellulose, alginate, gelatin, polyvinyl pyrrolidone, sucrose, and Arabic gum; (c) humectants, e.g., glycerol; (d) disintegrants, e.g., agar, calcium carbonate, potato starch or tapioca starch, alginate, and certain complex silicates ) humectants, e.g., glycerol; (d) disintegrants, e.g., agar, calcium carbonate, potato starch or cassava starch, alginic acid, certain composite silicates, and sodium carbonate; (e) retarding solvents, e.g., paraffin; (f) absorption accelerators, e.g., quaternary ammonium compounds; (g) wetting agents, e.g., cetyl alcohol and glycerin monostearate; (h) adsorbents, e.g., kaolin; and (i) lubricants, e.g. , talc, calcium stearate, magnesium stearate, solid polyethylene glycol, sodium dodecyl sulfate, or mixtures thereof. Among capsules, tablets and pills, the dosage forms may also contain buffers.

Solid dosage forms, such as tablets, sugared pills, capsules, pills and granules, may be prepared from coatings and shell materials, such as enteric coats and other materials well known in the art. They may contain opacifying agents, and active compounds or compounds in such compositions may be released in a delayed manner in a portion of the digestive tract. Examples of available embedding components are polymeric substances and waxy substances. The active compound may also be formed into a microencapsulated form with one or more of the above-mentioned excipients as necessary.

Liquid dosage forms for oral administration include pharmaceutically acceptable emulsions, solutions, suspensions, syrups or tinctures. In addition to the active compounds, the liquid dosage forms may contain inert diluents conventionally used in the art, such as water or other solvents, solubilizers and emulsifiers, for example, ethanol, isopropanol, ethyl carbonate, ethyl acetate, propylene glycol, 1,3-butylene glycol, dimethylformamide and oils, particularly cottonseed oil, peanut oil, maize embryo oil, olive oil, castor oil and sesame oil, or mixtures of these substances and the like.

In addition to these inert diluents, the compositions may also contain additives, such as wetting agents, emulsifiers and suspending agents, sweeteners, corrective agents and fragrances.

In addition to the active compounds, the suspensions may contain suspending agents, such as ethoxylated isooctadecanol, polyoxyethylene sorbitol and dehydrated sorbitan ester, microcrystalline cellulose, aluminum methoxide and agar or mixtures of these substances and the like.

Compositions for parenteral injection may contain physiologically acceptable sterile aqueous or anhydrous solutions, dispersions, suspensions or emulsions, and sterile powders for redissolution into sterile injectable solutions or dispersions. Appropriate aqueous and non-aqueous carriers, diluents, solvents or excipients include water, ethanol, polyols and appropriate mixtures thereof.

Dosage forms of the compounds of the present invention for topical administration include ointments, powders, patches, spraying agents and inhalants. Under sterile conditions, the active ingredient is mixed together with a physiologically acceptable carrier and any preservatives, buffers, or, if necessary, propellants as potentially required.

The compounds of the present invention may be administered alone, or in combination with other pharmaceutically acceptable compounds.

Use of a pharmaceutical composition means that a safe and effective amount of the compounds of the present invention is administered to a mammal (e.g., a human) to be treated, wherein the dose at the time of administration is an effective dosage of administration in a pharmaceutical sense, and the daily dosage of administration is typically 1-2,000 mg, preferably 20-500 mg, for a human with a body weight of 60 kg. Of course, the specific dosage should also take into account factors, such as the route of administration and the health condition of a patient, which are all within the skill of a skilled physician.

### Composition or formulation, combination of active ingredients and kit and methods of administration

The present invention also provides a composition or formulation, a combination of active ingredients and a kit, wherein the composition or formulation, combination of active ingredients and kit can be used to prepare an innate immune-activating drug including: a) a broad-spectrum antiviral drug; b) an anti-tumor drug; c) a drug for preventing and/or treating cancer; d) a drug for treating or preventing type I interferon-related diseases; e) an immune adjuvant; and/or (f) a cytokine inducer.

The compositions described herein are preferably pharmaceutical compositions. The compositions described herein may include pharmaceutically acceptable carriers.

The pharmaceutical formulation should be matched with the mode of administration, preferably oral administration, injectable administration (e.g., intratumoral injection), and when used, a therapeutically effective amount of drug is administered to a desired subject (e.g., a human or a non-human mammal). The term "therapeutically effective amount" as used herein refers to an amount that is functional or active in humans and/or animals and acceptable to humans and/or animals. It should be understood by those of ordinary skill in the art that the "therapeutically effective amount" may vary with the form of a pharmaceutical composition, the route of administration, the excipients of a drug used, the severity of a disease, medication in combination with other drugs and the like.

In one mode of administration, a safe and effective daily application dosage of the first active ingredient is typically at least about 0.1 mg, and not more than about 2,500 mg in most cases. Preferably, this dosage is 1 mg-500 mg; the safe and effective amount of the second active ingredient is typically at least about 0.01 mg and not more than 2,500 mg in most cases. Preferably, the dosage ranges from 0.1 mg to 2,500 mg. Of course, the specific dosage should also take into account factors, such as the route of administration and the health condition of a patient, which are all within the skill of a skilled physician.

### Example 1 Secretion of interferon in cells induced by compounds of the present invention

Firstly, the following 11 newly synthesized small molecule compounds (LD001 - LD011) were tested for activity in inducing generation of type I interferon in different types of cells, such as HEK293T, HeLa, A549 and PEM.

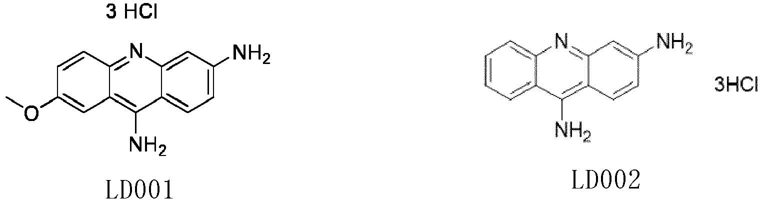

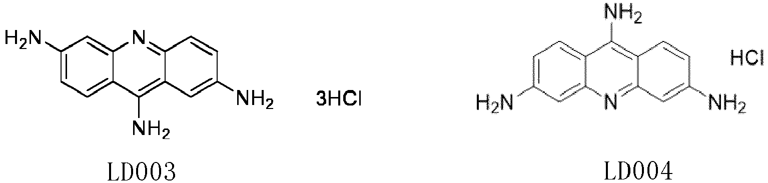

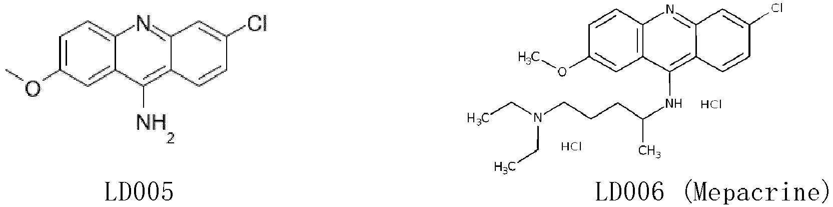

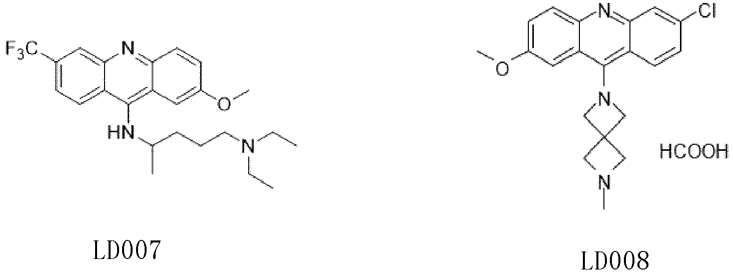

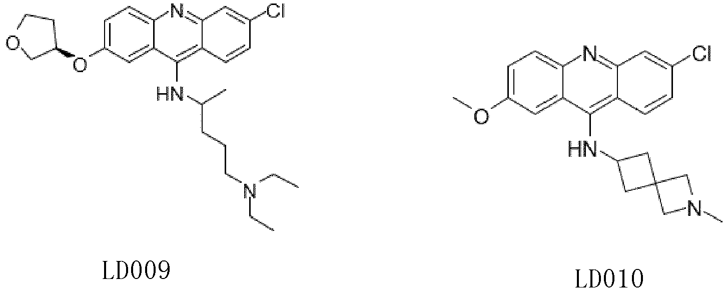

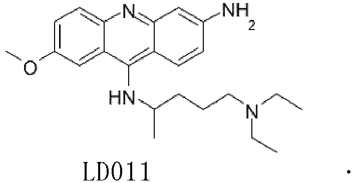

The 293T cell line, HeLa cell line and A549 cell line used were all purchased from Cell Bank of Shanghai Institutes for Biological Sciences, Chinese Academy of Sciences. Cell monolayers were cultured at 37 °C in a 5% CO₂ incubator, and cells grew in DMEM medium containing 10% FBS and 100 U/ml penicillin and 100 µg/ml streptomycin. PEM cells were macrophages isolated from the peritoneal cavity of C57BL/6 mice.

Various cells, such as HEK293T, HeLa, A549 and PEM cells in logarithmic growth phase, were spread in 12-well cell culture plates at an inoculum density of 80-90% respectively; after the cells had been attached to the wall for 6 h, the cell culture supernatant was replaced with a medium containing the various small molecule compounds LD001-LD011 with a final concentration of 10 µM, the cells continued to be cultured for 12 h and finally collected, and the total cellular RNA was extracted.

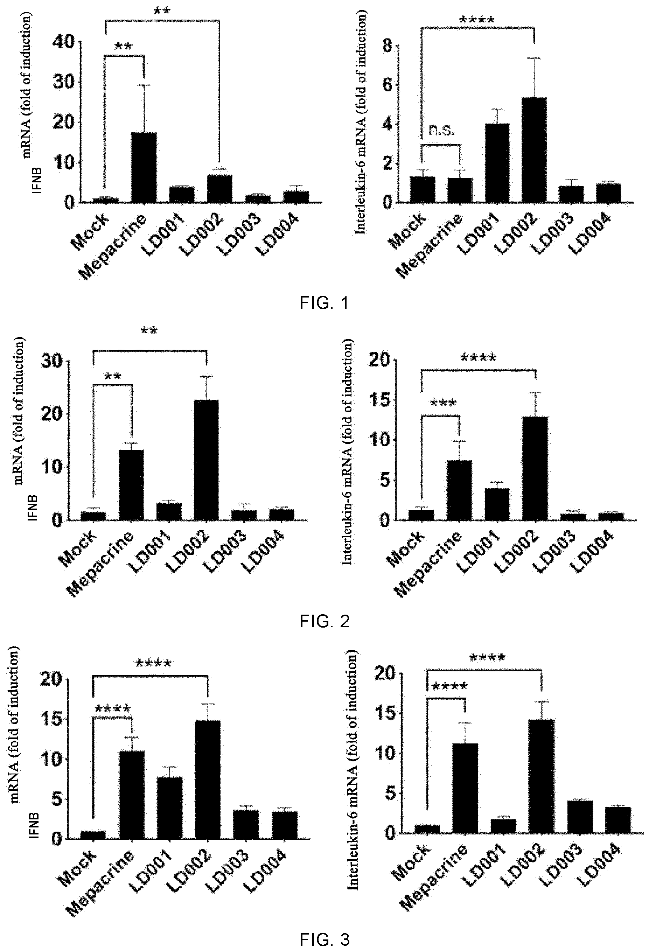

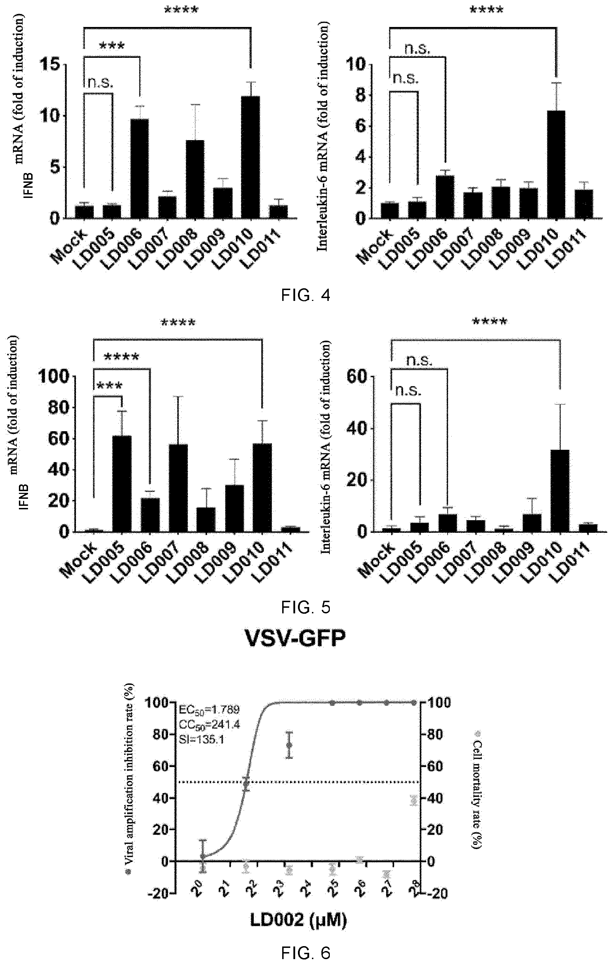

Next, the transcriptional activation of type I interferon and interleukin-6 induced by various cells under drug treatment was detected by qPCR, and the results showed that LD002 could promote the transcription of type I interferon in three kinds of cells, i.e., HEK293T, HeLa and A549, more significantly, and the transcriptional level of interleukin-6 was also elevated to a certain extent (FIG. 1, FIG. 2 and FIG. 3). In addition, the results showed that LD007 and LD010 could remarkably induce expression of type I interferon in HEK293T and PEM cells, and LD005 could significantly induce expression of type I interferon in PEM cells (FIG. 4 and FIG. 5). In conclusion, it will be seen from FIGS. 1-5 that the compounds LD001-LD011 have the activity of inducing expression of type I interferon or interleukin-6 in HEK293T, HeLa, A549 or PEM cells. In view of the fact that LD007 and LD010 have inhibiting effects on cell growth and exhibit strong cytotoxicity, and that LD005 has low solubility in PBS, compound LD002 was selected as a representative compound in the present invention for research on subsequent embodiments.

### Example 2 Inhibition of RNA viruses (VSV and H1N1-IAV) by compound LD002 of the present invention

The results of Example 1 above have demonstrated that LD002 could induce the expression of type I interferon in cells more significantly, and next, the inhibiting effect of LD002 on virus amplification was detected by a cellular model of virus infection. Vesicular stomatitis virus (VSV) and influenza virus (IAV) were selected as representatives of RNA viruses; specifically, VSV-GFP and H1N1 virus strains were used, and VSV-GFP itself was capable of expressing green fluorescent protein GFP, which facilitated indication of viral proliferation and load.

A549 cells in logarithmic growth phase were spread in 12-well cell culture plates at an inoculum density of 80-90%; after the cells had been attached to the wall for 6 hours, a dilution of VSV with a multiplicity of infection (MOI) of 0.5 was added. The cells were treated with different concentrations of the compound LD002 of the present invention one hour after virus infection, and whole-cell RNA extraction and detection of virus amplification were performed after 12 hours. Based on the extent of inhibition of VSV-GFP infection after treatment of A549 cells with different concentrations of LD002, the EC₅₀ (Median Effective Concentration) of the inhibiting effect of LD002 on VSV-GFP infection was calculated to be 1.789, the SI (Selection Index) was 135.1 (FIG. 6), and the results showed that the compounds of the present invention had excellent anti-VSV activity and safety.

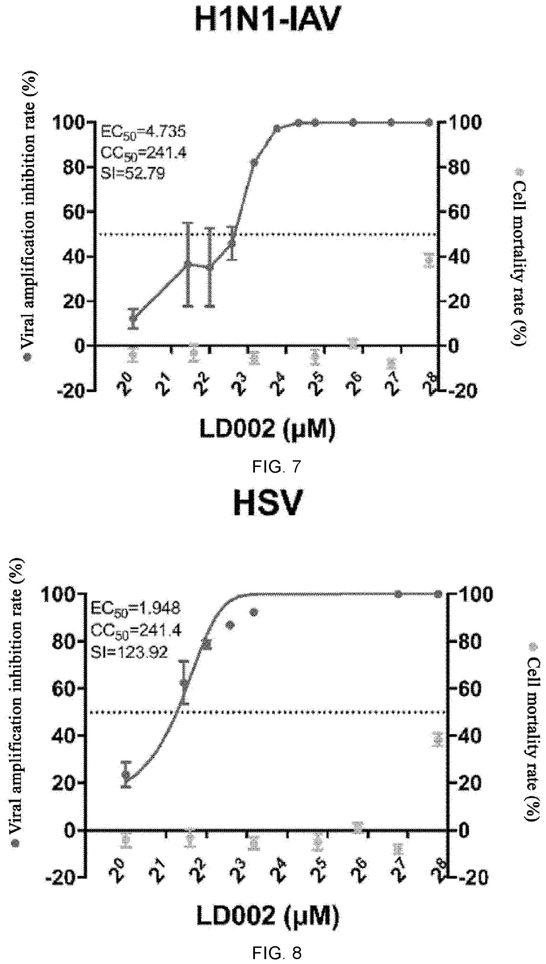

Similarly, A549 cells in logarithmic growth phase were spread in 12-well cell culture plates at an inoculum density of 80-90%; after the cells had been attached to the wall for 6 hours, a dilution of H1N1-IAV with a multiplicity of infection (MOI) of 0.5 was added. The cells were treated with different concentrations of the compound LD002 of the present invention one hour after virus infection, and whole-cell RNA extraction and detection of virus amplification were performed after 12 hours. Based on the extent of inhibition of H1N1-IAV infection after treatment of A549 cells with different concentrations of LD002, the EC₅₀ (Median Effective Concentration) of the inhibiting effect of LD002 on H1N1-IAV infection was calculated to be 4.735, the SI (Selection Index) was 52.79 (FIG. 7), and the results showed that the compounds of the present invention had better anti-IAV activity and safety.

### Example 3 Inhibiting effect of compound LD002 of the present invention on DNA viruses (such as HSV)

Herpes Simplex Virus (HSV) was selected as a representative of DNA viruses; specifically, an HSV-1-GFP virus strain was used and capable of expressing the green fluorescent protein (GFP) by itself and indicating the viral proliferation and load.

A549 cells in logarithmic growth phase were spread in 12-well cell culture plates at an inoculum density of 80-90%; after the cells had been attached to the wall for 6 hours, a dilution of the HSV with a multiplicity of infection (MOI) of 0.5 was added. The cells were treated with different concentrations of the compound LD002 of the present invention one hour after virus infection, and whole-cell RNA extraction and detection of virus amplification were performed after 12 hours. Based on the extent of inhibition of HSV-GFP infection after treatment of A549 cells with different concentrations of LD002, the EC₅₀ (Median Effective Concentration) of the inhibiting effect of LD002 on HSV-GFP infection was calculated to be 1.948, the SI (Selection Index) was 123.92 (FIG. 8), and the results showed that the compounds of the present invention had excellent anti-HSV activity and safety.

### Example 4 Induction of interferon generation in mice in vivo with the compound LD002 of the present invention

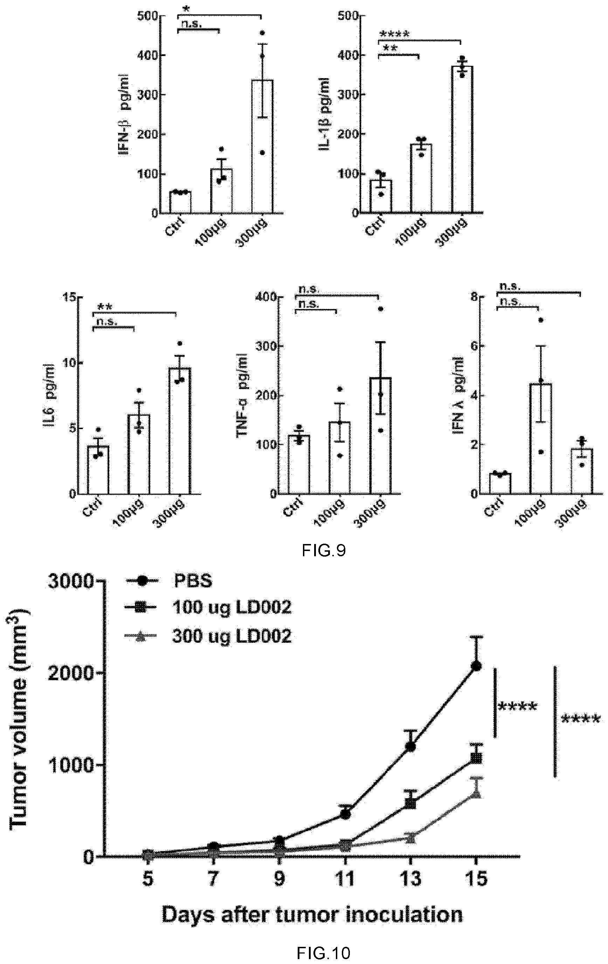

The mice used in all experiments were 6 to 8-week-old male C57BL/6 mice provided by the Animal Experiment Technology Platform of the Center for Excellence in Molecular Cell Science. The mice were injected intraperitoneally with different doses (100 µg or 300 µg) of the compound LD002 of the present invention, and changes in contents of various cytokines in serum were detected by ELISA after 6 hours. The results showed that an experimental group of the compound LD002 of the present invention could remarkably increase the levels of type I interferon IFNβ (interferon-β) and IL1β (interleukin-1β) in serum, and could slightly increase the expression levels of IL-6 (interleukin-6), TNFα (tumor necrosis factor-α) and type III interferon IFNλ (interferon-λ) (FIG. 9). In FIG. 9, Ctrl is a control group injected intraperitoneally with PBS at an injected dose of 100 µL/mouse.

### Example 5 Compound LD002 of the present invention has an anti-tumor effect and a function of promoting tumor immunotherapy

### 1. Method

B16-F10 melanoma cell strains used in this experiment were purchased from the cell bank of Chinese Academy of Sciences Shanghai Branch, conventionally cultured in a DMEM medium containing 10% FBS, and placed in a 5% CO₂ incubator for culturing at 37°C. Cells in the exponential proliferation phase were digested with 0.25% trypsin, centrifuged, resuspended in PBS and counted, the cell survival rate was detected to be above 95%, and the concentration of cell suspension was adjusted to 2*10^⁶ mL⁻¹ after viable cells were counted. Resuspended tumor cells were dispensed into sterile EP tubes and placed on ice for later use.

The hair on the backs of the mice was removed with an electric shaver, and the hair removal area was about 9 cm². The prepared B16-F10 cells mentioned above were taken, and 100 µL of tumor cell suspension was injected subcutaneously into the back of each mouse. Tumor growth in the mice was observed on a daily basis, the formation of melanoma was visible to the naked eye on Day 4, mice in which no tumor had been formed were excluded, the remaining mice were divided into 7 groups randomly, and the number of mice in each experimental group was five.

Groups in Experiment I respectivelyincluded a control group, a group of 100 µg of the compound LD002 of the present invention, and a group of 300 µg of the compound LD002 of the present invention; groups in Experiment II respectively included a control group, a group of 100 µg of the compound LD002 of the present invention, a group of 200 µg of the anti-PD-L1 antibody (B7-H1) (Clone: 10F.9G2), a group of 100 µg of the compound LD002 of the present invention + a group of 200 µg of the anti-PD-L1 antibody (B7-H1) (Clone: 10F.9G2), with 6-8 mice in each group. The experimental method was that: administration to tumor-bearing mice was performed on Day 5 after tumor cell injection based on the groups, LD002 was injected intraperitoneally, and two dose groups were set: 100 µg/mouse and 300 µg/mouse. An anti-PD-L1 antibody was administered by intraperitoneal injection, and one dose group was set: 200 µg/mouse. The solvent-buffer system for administration was a PBS buffer solution. The same volume of PBS buffer solution was administered to the control groups by gavage. For the compound drugs of the present invention, each mouse was administered once every 2 days for a total of 3 doses. The administration volume was 100 µL. For the anti-PD-L1 antibody, each mouse was administered once every 2 days for a total of 3 doses. The administration volume was 50 µL. Specifically, the administration was performed on Day 5, Day 7 and Day 9, respectively at an interval of 2 days for a total of 3 doses. Tumor sizes were measured every other day, survival periods of the mice were observed, related data were recorded, tumor volumes were measured with electronic calipers, and the volume was calculated by the formula of π/6×length×width×height. When the tumor volume was greater than 2,000 mm³, the tumor-bearing mice were killed.

### 2. Experimental results

The compound LD002 of the present invention inhibited the growth of subcutaneous melanoma in mice and extended the survival period of the tumor-bearing mice.

By establishing a model of the tumor-bearing mice with subcutaneous melanoma, the inhibiting effect of the compound LD002 of the present invention and LD002 + anti-PD-L1 on the growth of tumors was detected, and the survival period of the tumor-bearing mice was extended. The results showed that the administration of the compounds of the present invention alone significantly inhibited the growth of melanoma in a dose-dependent manner and extended the survival period of the tumor-bearing mice compared with the control groups (FIG. 10).

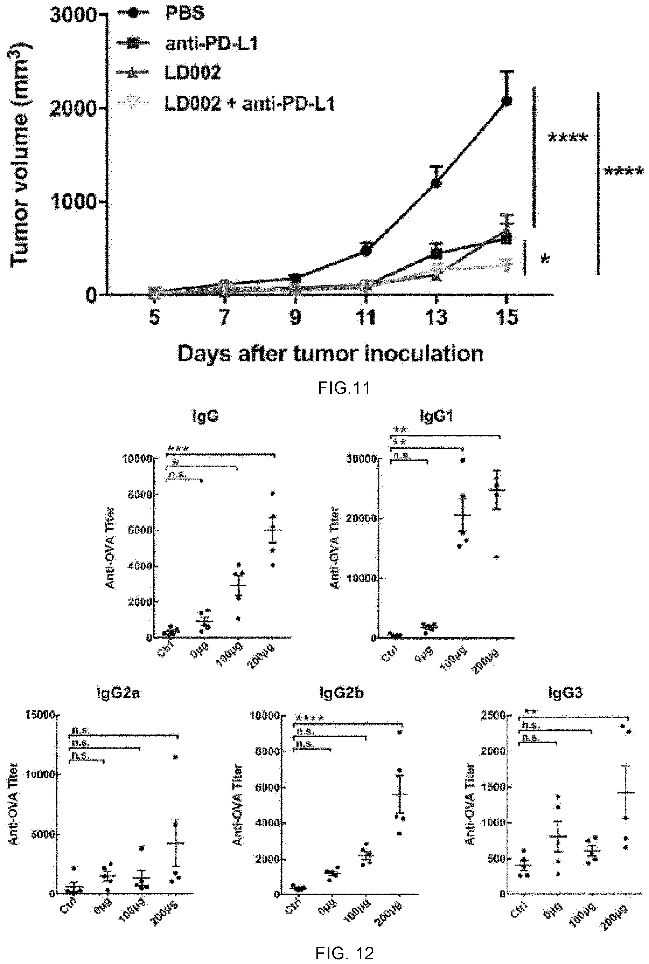

The Anti-PD-L1 was capable of significantly enhancing the inhibiting effect of the compounds of the present invention on melanoma growth and extending the survival period of the mice compared with the control groups and administration of the compound LD002 of the present invention alone. It can be seen from the resulting data that the compounds LD002 of the present invention and anti-PD-L1 were capable of exerting synergistic effects jointly, having a more significant inhibiting effect on melanoma growth, and extending the survival period of the mice (FIG. 11).

It can be seen from Examples 4 and 5 that the compound LD002 of the present invention was capable of inducing an increase in various cytokines, such as interferon, in the peripheral blood, having an excellent inhibiting effect on tumors, such as melanoma, and extending the survival period. The combination of the compound LD002 of the present invention and anti-PD-L1 had a synergistic effect in inhibiting tumor growth and extending the survival period. Thus, the compound of the present invention is capable of functioning as a drug which induces various cytokines, such as interferon, and has an anti-tumor effect and a function of promoting tumor immunotherapy.

### Example 6 Compound LD002 of the present invention has immune adjuvant activity

The experiment was divided into a blank group (injected with the same volume of PBS), an antigen group and an antigen group/a group of the compound of the present invention (containing different concentration gradients), with 5 mice in each group. Antigen ovalbumin (OVA) and the compound LD002 of the present invention were dissolved in PBS respectively to obtain an antigen solution and a solution of the compound of the present invention, and solutions containing the antigen and the compound of the present invention with different concentration gradients were prepared according to concentration requirements. Each mouse in the experimental group was immunized with 100 µl of antigen solution or antigen solution and LD002 solution each time by intramuscular injection. Mice in the blank group were injected with the same volume of PBS solution as the experimental group. The first immunization was set to be performed on Day 0, and was enhanced once on Day 7 and Day 14, respectively. After immunization on Day 21, blood was collected from the orbits of the mice, serum was collected from the mice and diluted, and the diluted serum was tested for an antibody content in the serum of the mice by ELISA. The results showed that OVA alone did not generate a protective humoral immune response after immunization compared with the antigen group, and when the mice were immunized with OVA combined with the compound of the present invention, the total amount of OVA-specific IgG, IgG1 and IgG2b antibodies could be significantly increased, indicating that the compound of the present invention had remarkable immune adjuvant activity (FIG. 12).

The present invention will be further described below in conjunction with specific embodiments. It should be understood that these embodiments are used only to illustrate the present invention, but not intended to limit the scope of the present invention. Experimental methods for which specific conditions are not indicated in the following embodiments are usually in accordance with conventional conditions, or conditions recommended by the manufacturer. Unless otherwise specified, percentages and parts shall be calculated by weight.

## Claims

1. Use of a compound of formula I or a tautomer, a mesomer, a racemate, an enantiomer, a diastereoisomer or a mixture thereof, or a pharmaceutically acceptable salt thereof, or a prodrug thereof for preparing an innate immune-activating drug, 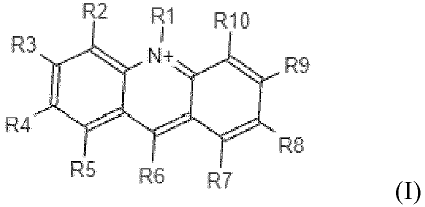 wherein R1, R2, R3, R4, R5, R6, R7, R8, R9 and R10 are each independently selected from hydrogen, halogen, alkyl, cycloalkyl, aryl, heteroaryl, heterocyclyl, alkenyl, alkynyl, amino, hydroxy, hydrosulfuryl, carboxy, alkoxy, cycloalkoxy, haloalkyl, cyano, thioalkyl, sulpho, sulfuryl, sulfoxide group and phosphate group, and the alkyl, cycloalkyl, alkoxy, cycloalkoxy, amino, heterocyclyl, aryl or heteroaryl may be substituted with one or more substituents selected from one or more of hydroxyl, halogen, alkyl, alkoxy, cycloalkyl, cycloalkoxy, heterocyclyl, aryl, haloaryl, haloalkyl, heteroaryl, alkenyl, alkynyl, amino, sulfydryl, carboxy, ester group, alkoxycarbonyl, acyloxy, acylamino, carbamido, alkylsulfonyl, aryl sulfonyl, cyano, nitro, nitroso, thiocyanato, isothiocyanato, thioalkyl, sulfonic group, phosphoryl group, phosphonic acid group, alkyl phosphate group, alkyl phosphonic acid group, aryl phosphate group and aryl phosphonic acid group, or null; the heterocyclyl comprises at least one N atom, or comprises 1 or 2 or 3 heteroatoms selected from N, S and O.

2. The use of claim 1, wherein the compound of formula I comprises:

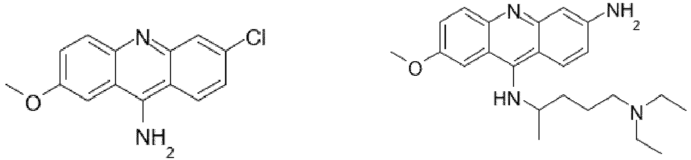

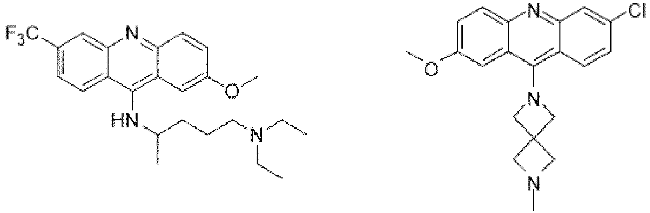

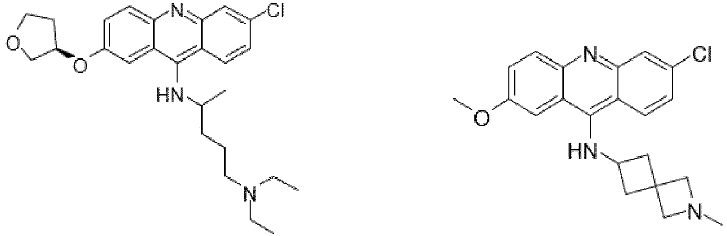

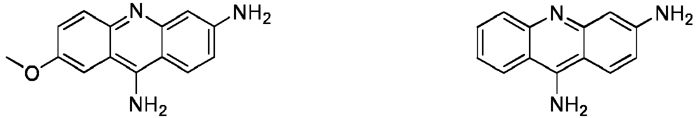

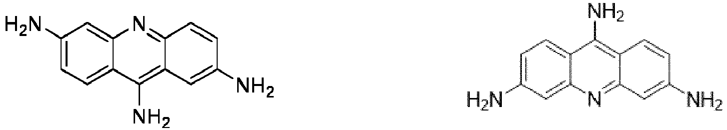

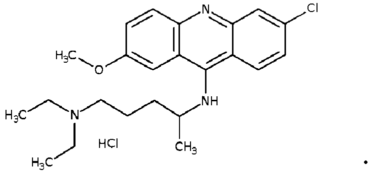

3. The use of claim 1 or 2, wherein the pharmaceutically acceptable salt of the compound of formula I comprises:

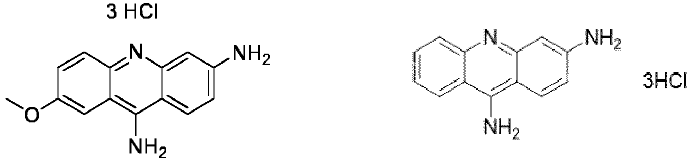

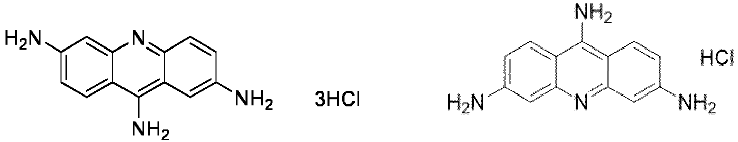

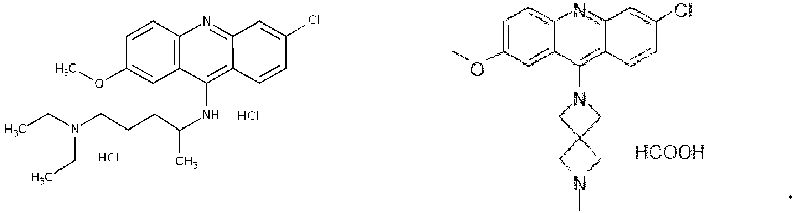

4. The use of claim 1, wherein the innate immune-activating drug comprises:
(a) a broad-spectrum antiviral drug;
(b) an anti-tumor drug;
(c) a drug for preventing and/or treating cancer;
(d) a drug for treating or preventing type I interferon-related diseases;
(e) an immune adjuvant; and/or
(f) a cytokine inducer.

5. The use of claim 4, wherein the immune adjuvant is used to prepare antibodies, vaccines, immunotherapeutic drugs and/or immune activators.

6. A broad-spectrum antiviral pharmaceutical composition, wherein the pharmaceutical composition comprises: the compound of formula I of claim 1 or 2 or a tautomer, a mesomer, a racemate, an enantiomer, a diastereoisomer or a mixture thereof, or a pharmaceutically acceptable salt thereof, or a prodrug thereof, and one or more pharmaceutically acceptable carriers, diluents or excipients.

7. An immune composition, wherein the immune composition comprises: the compound of formula I of claim 1 or 2 or a tautomer, a mesomer, a racemate, an enantiomer, a diastereoisomer or a mixture thereof, or a pharmaceutically acceptable salt thereof, or a prodrug thereof; and an antigen.

8. An anti-tumor pharmaceutical composition, wherein the pharmaceutical composition comprises: the compound of formula I of claim 1 or 2 or a tautomer, a mesomer, a racemate, an enantiomer, a diastereoisomer or a mixture thereof, or a pharmaceutically acceptable salt thereof, or a prodrug thereof, and one or more pharmaceutically acceptable carriers, diluents or excipients.

9. A composition, wherein the composition comprises:
(a) a first active ingredient, which is a compound of formula I or a tautomer, a mesomer, a racemate, an enantiomer, a diastereoisomer or a mixture thereof, or a pharmaceutically acceptable salt thereof, or a prodrug thereof; and
(b) a therapeutically effective amount of second active ingredient, which comprises: an immunotherapeutic activator, a type I interferon modulator, or an antiviral agent.

10. A kit, wherein the kit comprises:
(A) a first formulation containing a first active ingredient which is a compound of formula I or a tautomer, a mesomer, a racemate, an enantiomer, a diastereoisomer or a mixture thereof, or a pharmaceutically acceptable salt thereof, or a prodrug thereof; and
(B) a second formulation containing a second active ingredient which comprises: an immunotherapeutic activator, a type I interferon modulator, or an antiviral agent.

11. Use of the composition of claim 9 or of the kit of claim 10, wherein the composition or the kit is used to prepare an innate immune-activating drug comprising:
(a) a broad-spectrum antiviral drug;
(b) an anti-tumor drug;
(c) a drug for preventing and/or treating cancer;
(d) a drug for treating or preventing type I interferon-related diseases;
(e) an immune adjuvant; and/or
(f) a cytokine inducer.

12. A compound of formula I or a tautomer, a mesomer, a racemate, an enantiomer, a diastereoisomer or a mixture thereof, or a pharmaceutically acceptable salt thereof, or a prodrug thereof, 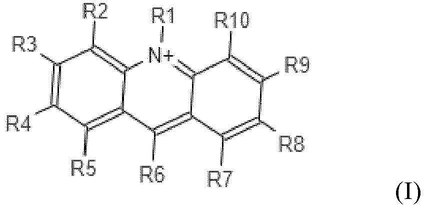 wherein R1, R2, R3, R4, R5, R6, R7, R8, R9 and R10 are each independently selected from hydrogen, halogen, alkyl, cycloalkyl, aryl, heteroaryl, heterocyclyl, alkenyl, alkynyl, amino, hydroxy, hydrosulfuryl, carboxy, alkoxy, cycloalkoxy, haloalkyl, cyano, thioalkyl, sulpho, sulfuryl, sulfoxide group and phosphate group, and the alkyl, cycloalkyl, alkoxy, cycloalkoxy, amino, heterocyclyl, aryl or heteroaryl may be substituted with one or more substituents selected from one or more of hydroxyl, halogen, alkyl, alkoxy, cycloalkyl, cycloalkoxy, heterocyclyl, aryl, haloaryl, haloalkyl, heteroaryl, alkenyl, alkynyl, amino, sulfydryl, carboxy, ester group, alkoxycarbonyl, acyloxy, acylamino, carbamido, alkylsulfonyl, aryl sulfonyl, cyano, nitro, nitroso, thiocyanato, isothiocyanato, thioalkyl, sulfonic group, phosphoryl group, phosphonic acid group, alkyl phosphate group, alkyl phosphonic acid group, aryl phosphate group and aryl phosphonic acid group, or null; the heterocyclyl comprises at least one N atom, or comprises 1 or 2 or 3 heteroatoms selected from N, S and O.

13. The compound of claim 12, wherein the compound of formula I comprises:

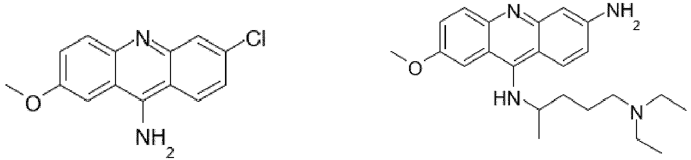

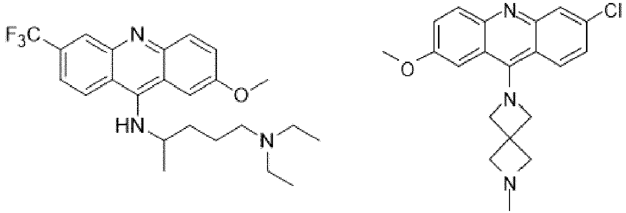

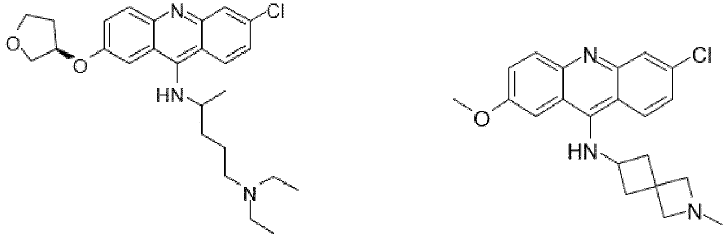

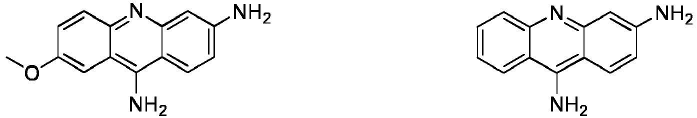

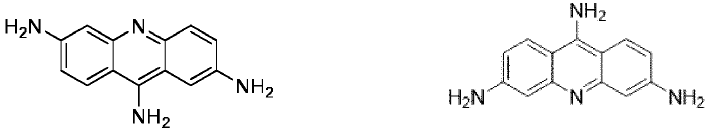

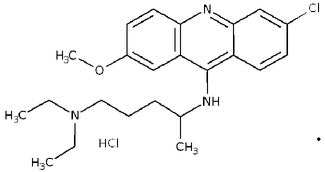

14. The compound of claim 12, wherein the pharmaceutically acceptable salt of the compound of formula I comprises:
